# EUROPEAN PATENT APPLICATION

(11) **EP 4 623 942 A1**
(43) Date of publication of application: **01.10.2025**
(21) Application number: 25167779.5
(22) Date of filing: 01.04.2025
(51) Int. Cl.: A61L 27/30, A61L 27/36

(54) **METHOD TO REDUCE THE CALCIFICATION OF A BIOLOGICAL HEART VALVE PROSTHESIS BY A TITANIUM CONTAINING COATING IN COMBINATION WITH UV IRRADIATION**

(30) Priority: 09.04.2024 IT 202400007861
(71) Applicant: pfm medical titanium gmbh, 90449 Nürnberg (DE)
(72) Inventor: Guldner, Norbert, 23627 Groß Sarau (DE); Maleika, Marek, 90766 Fürth (DE); Göller, Willi, 91522 Ansbach (DE)
(74) Representative: Lippert Stachow Patentanwälte Rechtsanwälte Part.mbB

(57) **Abstract**

The invention relates to a method to reduce the calcification of a biological heart valve prosthesis by a titanium containing coating in combination with UV irradiation. The method for treating the biological heart valve prosthesis with a titanium containing coating, particularly a glutaraldehyde-fixed pericardium, comprising the steps of:
a) treating the biological heart valve prosthesis with a titanium containing coating; and
b) irradiating the treated biological heart valve prosthesis with light of a wavelength between 100 nm and 450 nm.

This results in a biological heart valve prosthesis by a hypothesized polymerization with a homogenous surface.

## Description

The invention relates to a method to reduce the calcification of a biological heart valve prosthesis by a titanium containing coating in combination with UV irradiation, particularly to a method for treating a biological heart valve prosthesis with a titanium containing coating, particularly to a method for treating a glutaraldehyde-fixed pericardium as biological heart valve prosthesis with a titanium containing coating in order to reduce calcification.

If a heart valve is damaged or impeded, e.g. due to the effects of a stenosis caused by calcification, and can't be repaired by re-constructive procedures, for example using a catheter procedure called balloon valvuloplasty or other medical procedures, it finally must be replaced. In this case, the damaged heart valve is removed and replaced by a mechanical valve or a bioprosthetic heart valve made of a biological tissue valve, like bovine or porcine, particularly a bovine or porcine pericardium.

Usually valve replacement requires an open-heart procedure with a "sternotomy", in which the chest is surgically separated (opened) for the procedure. The transcatheter aortic valve implantation (TAVI) procedure or also referred to as transcatheter aortic valve replacement (TAVR) procedure can be done through a very small opening that leaves all the chest bones in place. The TAVI procedure is performed using one of two approaches, (i) Entering through the femoral artery (large artery in the groin), called the transfemoral approach, which does not require a surgical incision in the chest, or (ii) Using a minimally invasive surgical approach with a small incision in the chest and entering through a large artery in the chest or through the tip of the left ventricle (the apex), which is known as the transapical approach.

Another kind of heart valve replacement is the so-called Ross Procedure, where the diseased aortic valve is replaced with the patient's own pulmonary valve.

A mechanical heart valve is made of strong, durable materials and may last throughout a patient's life. However, patients receiving a mechanical heart valve usually require a blood-thinning medication (anticoagulation therapy) for the rest of their life. The blood thinner will keep clots from forming, which is critical because clots can lodge in the valve flaps or hinges and cause a malfunction. Clots can also break off and form into an embolism (traveling clot), which may move through the bloodstream and lodge into a vessel where it may lead to problems such as heart attack or stroke. On the other hand, blood thinner can have serious side-effects, particularly life-threatening bleedings (brain and stomach), and are only prescribed if absolutely necessary, particularly over a long period.

The biological tissue valves of a bioprosthetic heart valve are made from bovine or porcine tissue, like pericardium. They do not require chronic anticoagulation therapy. However, they degenerate prematurely, especially in young patients. It is generally accepted that the breakdown of bioprosthetic heart valves is due to calcification that occurs because of chemical processes and a retaining antigenicity, even after glutaraldehyde treatment. Calcium processes occur between free aldehyde groups and phospholipids, and in combination with calcium ions from the circulation, this induces calcification.

In the article 'Detoxification and Endothelialization of Glutaraldehyde-Fixed Bovine Pericardium With Titanium Coating' Norbert W. Guldner, et. al. (Circulation, 2009; 119:1653-1660) discuss the impact of a titanium coating on the biocompatibility and durability of glutaraldehyde-fixed cardiovascular bioimplants, particularly of glutaraldehyde-fixed pericardium.

In the article 'Nanocoating with titanium reduces iC3b- and granulocyte-activating immune response against glutaraldehyde-fixed bovine pericardium: A new technique to improve biological heart valve prosthesis durability?' Norbert W. Guldner, et. al. (The Journal of Thoracic and Cardiovascular Surgery 2012; 143:1152-9) discuss the ability of titanium nanocoating on glutaraldehyde-fixed bovine pericardium to prevent major immunoreactions.

In the article 'The first self-endothelialized titanium-coated glutaraldehyde-fixed heart valve prosthesis within systemic circulation' Norbert W. Guldner, et. al. (The Journal of Thoracic and Cardiovascular Surgery; July 2009; 248-250) discuss the impact of titanium nanocoating on glutaraldehyde-fixed porcine pericardium to support detoxification and endothelialization with less thrombo-embolic complications.

None of the afore-mentioned disclosures sufficiently solved the problem of a breakdown of bioprosthetic heart valves due to calcification over time.

It is therefore an object of the present invention to provide a method for treating a biological heart valve prosthesis to prevent calcification thereof over time.

The object is solved by the method according claim 1. Preferred embodiments of the invention are defined in the dependent claims. The invention further relates to a biological heart valve prosthesis, particularly a Transcatheter-Aortic-Valve-Implantation valve, treated by a method according to the invention.

The method according to the present invention for treating a biological heart valve prosthesis with a titanium containing coating, particularly a glutaraldehyde-fixed pericardium, comprises the steps of:
a) treating the biological heart valve prosthesis with a titanium containing coating; and
b) irradiating the treated biological heart valve prosthesis with light of a wavelength between 100 nm and 450 nm.

It has been observed that a biological heart valve prosthesis with a titanium containing coating is still subject to calcification over time. By spectroscopy, particularly scanning electron microscope, it was found that the titanium containing coating forms structural damages over time. Calcium particles can settle at these structural damages over time, resulting in a calcification of the biological heart valve prosthesis. After 9 weeks in a calcium tester the biological heart valve prosthesis treated by the inventive method showed 70 % less calcification compared to an untreated or only titanium containing coating biological heart valve prosthesis.

A biological heart valve prosthesis treated by the method according to the present invention has the advantage, that it can be implanted as a TAVI without an extra-corporal circulation and after implantation does not require any blood thinner and hence the risk of inner bleedings is significantly reduced.

It has been found out that irradiating the treated biological heart valve prosthesis with light of a wavelength between 100 nm and 450 nm significantly reduced the formation of structural damages and hence results in a major reduction in calcification of the biological heart valve prosthesis.

This observation was made by the inventors after they left an already titanium coated biological heart valve prosthesis unattended close to a window for several weeks and only afterwards integrated this particular biological heart valve prosthesis into a calcification with other recently titanium coated biological heart valve prosthesis. This particular biological heart valve prosthesis was significantly less calcified compared to the recently titanium coated biological heart valve prosthesis. After further examination and experiments, it has been found out that irradiating the treated biological heart valve prosthesis with light of a wavelength between 100 nm and 450 nm results in reduced formation of structural damages and hence less calcification. The non-radiated valve ruptured and became insufficient. Hypothesis is that irradiation with light of a wavelength between 100 nm and 450 nm polymerizes the blood contacting surface with nearly no structural damages.

According to a variant of the invention, the method further comprises the step of dehydrating the biological heart valve prosthesis before step a). The step of dehydrating the biological heart valve prosthesis before coating step a) is beneficial for the coating process and the bounding of the titanium atoms to the biological heart valve prosthesis. The step of dehydrating the biological heart valve prosthesis is for example performed for 2 hours at 35°C under vacuum or low pressure.

Pursuant to a variant of the invention, the method comprises the step of rehydrating the coated biological heart valve prosthesis before or after step b). Particularly, the coated biological heart valve prosthesis is rehydrated by storing the coated biological heart valve prosthesis in a water-based organic solvent. For example, the dehydrated, coated and irradiated biological heart valve prosthesis is rehydrated for 30 minutes in a physiological saline solution (0.9 %).

According to a preferred variant of the invention, the method comprises the step of coating the biological heart valve prosthesis with a polysiloxane, particularly before step a). For example, the biological heart valve prosthesis is wet chemical coated with Bis(triethoxysilyl)R; R = Methane, Ethane, Propane, Butane, Pentane, Hexane, Heptane, Octane, Nonane, Decane, Undecane, Dodecane, Carbonate, Ethylene. The irradiation of the treated biological heart valve prosthesis with light of a wavelength between 100 nm and 450 nm causes a polymerization of polysiloxane and thereby further reduces the calcification of biological heart valve prosthesis.

Pursuant to a variant of the invention, the biological heart valve prosthesis is stored during step b) in an organic solvent. For example, the organic solvent is ethyl-alcohol with at least 80 %.

In a variant of the invention, the step a) bases on a wet-chemical coating process, a physical vapor deposition (PVD), sputtering coating process or a plasma-activated chemical vapor deposition process.

According to a preferred variant of the invention, step a) bases on a plasma-activated chemical vapor deposition process comprising a pre-processing step and a coating step. Such a plasma-activated chemical vapor deposition is a coating technology that for example transfers the so-called precursor tetrakis(dimethylamido)titanium {Ti[N(CH₃)₂]₄} into the gas phase and brings it into the reactor by a carrier gas, such as nitrogen or hydrogen gas, under vacuum. Within nonthermal plasma with high electron temperatures but with neutrons and ions at room temperature, only the electrons can follow a quickly changing electrical field with a typical frequency of 40 kHz, 400 kHz or 13.56 MHz. The plasma is able to supply the precursor with high energy, while the temperature during deposition can be kept low, at approximately 30°C to 35°C. Under these conditions, the precursor is deconstructed, and parts of the precursor are precipitated on the plasma-activated biological heart valve prosthesis. The carbon atoms that are in contact with the titanium are then able to perform paired electron bindings with the carbon atoms of other ligands that have titanium bindings and with the carbon atoms of the biological heart valve prosthesis. Thus, the titanium containing coating is bound to the biological heart valve prosthesis in an extremely thin layer of about 30 nm.

Pursuant to a variant of the invention, the pre-processing step uses a plasma power between 100 Watt to 300 Watt and the coating step uses a plasma power between 100 Watt to 500 Watt. Particularly, the pre-processing step uses a plasma power of 200 Watt and the coating step uses a plasma power of 300 Watt.

In an advantageous variant of the invention, the plasma-activated chemical vapor deposition process comprises at least two cycles.

According to a further variant of the invention, each cycle has an ON period and an OFF period, particularly an ON period between 1 to 10 seconds and an OFF period between 10 and 30 seconds.

Pursuant to a preferred variant of the invention, step b) is performed for less than 72 hours, particularly less than 48 hours and preferably 24 hours or less.

In an expedient variant of the invention, step b) is performed using a light source with a power between 20 Watt and 100 Watt, particularly with a power between 30 Watt and 50 Watt. The power of the light source in combination with the distance to the biological heart valve prosthesis during irradiation are chosen to avoid any heat damage to the biological heart valve prosthesis. Preferably, the distance between the light source and the biological heart valve prosthesis is between 10 cm and 50 cm during irradiation, particularly between 15 cm and 20 cm. Advantageously, the biological heart valve prosthesis is placed in an irradiation chamber during irradiation by the light source, wherein the inner walls of irradiation chamber are reflective regarding the light irradiated by the light source, i.e. in the range of 100 nm and 450 nm.

According to a variant of the invention, the wavelength during step b) is between 190 and 380 nm.

Pursuant to an advantageous variant of the invention, step a) creates a titanium containing coating with a thickness of less than 200 nm, particularly with a thickness of less than 100 nm, further preferred with a thickness of less than 50 nm, on the biological heart valve prosthesis.

In a preferred variant of the invention, step a) is performed at temperatures below 50°C, particularly below 40°C. This prevents that the biological heart valve prosthesis is damaged by heat during step a).

The invention further relates to a biological heart valve prosthesis, particularly in Transcatheter-Aortic-Valve-Implantation (TAVI) valves, treated by a method according to the invention. Such a TAVI valve can be implanted by inserting a catheter in an arterial punction (arterial femoralis). Due to the method according to the present invention the calcification of such a TAVI valve is significantly reduced and the lifetime of the TAVI valve is increased. This increases the time-interval in which such TAVI valves must be replaced by many years.

In the following the invention is further explained with respect to a specific embodiment of the invention.

A bovine pericardium sold under the name "XenoSure" by company LeMaitre has been used for representing the biological heart valve prothesis. The bovine pericardium with the overall dimensions of 10 cm to 16 cm and a thickness between 0.35 mm and 0.75 mm has been cut to several probes having a circular shape and a diameter of 38 mm.

The circular probes represent the biological heart valve prosthesis in this embodiment. The probes have been stored in 0.2 % glutaraldehyde solution, so that the pericardium is glutaraldehyde fixed.

In the next step the probes have been coated with a polysiloxane. Particularly, the probes have been wet chemical coated with Bis(triethoxysilyl)octane.

Afterwards, the probes have been dehydrated for 2 hours at 35°C and under vacuum.

The dehydrated probes have been coated with titanium in a plasma-activated chemical vapor deposition process. The plasma-activated chemical vapor deposition process comprised a pre-processing step and a coating step, wherein the pre-processing step used a plasma power of 200 Watt and the coating step used a plasma power of 300 Watt. Furthermore, the plasma-activated chemical vapor deposition process comprised two cycles, wherein each cycle has an ON period and an OFF period. The ON cycle had a length of 2 seconds and the OFF cycle a length of 15 seconds. The temperature during this step was below 40°C. The thickness of the resulting titanium coating was less than 100 nm.

The coated probes have been stored in an 80 % ethanol solution (EtOH), wherein the remaining 20 % are preferably water.

Next, the probes have been irradiated with a light having a wavelength in the range between 300 nm and 400 nm, with a main wave peak for 365 nm. The power of the light source was 40 Watt and the probes have been irradiated for 24 hours. During this step, the probes have still been stored in the organic solvent of the previous step, i.e. 80 % ethanol solution.

## Claims

1. Method for treating a biological heart valve prosthesis with a titanium containing coating, particularly a glutaraldehyde-fixed pericardium, comprising the steps of:
a) treating the biological heart valve prosthesis with a titanium containing coating; and
b) irradiating the treated biological heart valve prosthesis with light of a wavelength between 100 nm and 450 nm.

2. Method according to claim 1,
further comprising the step of dehydrating the biological heart valve prosthesis before step a).

3. Method according to claim 2,
comprising the step of rehydrating the coated biological heart valve prosthesis before or after step b).

4. Method according to claim 3, wherein the coated biological heart valve prosthesis is rehydrated by storing the coated biological heart valve prosthesis in a water-based organic solvent

5. Method according to any of claims 1 to 4,
wherein the biological heart valve prosthesis is stored during step b) in an organic solvent.

6. Method according to any of claims 1 to 5,
comprising the step of coating the biological heart valve prosthesis with a polysiloxane, particularly before step a).

7. Method according to any of claims 1 to 6,
wherein the step a) bases on a wet-chemical coating process, a physical vapor deposition (PVD) sputtering coating process or a plasma-activated chemical vapor deposition process.

8. Metod according to claim 7,
wherein step a) bases on a plasma-activated chemical vapor deposition process comprising a pre-processing step and a coating step.

9. Method according to claim 8,
wherein the pre-processing step uses a plasma power between 100 Watt to 300 Watt and the coating step uses a plasma power between 100 Watt to 500 Watt.

10. Method according to claim 8 or claim 9,
wherein the plasma-activated chemical vapor deposition process comprises at least two cycles.

11. Method according to claim 10,
wherein each cycle has an ON period and an OFF period, particularly on ON period between 1 to 10 seconds and an OFF period between 10 and 30 seconds.

12. Method according to any of claims 1 to 11,
wherein step b) is performed for less than 72 hours.

13. Method according to any of claims 1 to 12,
wherein during step b) the wavelength is between 190 and 380 nm.

14. Method according to any of claims 1 to 13,
wherein step a) creates a titanium coating with a thickness of less than 200 nm, particularly with a thickness of less than 100 nm, further preferred with a thickness of less than 50 nm, on the biological heart valve prosthesis.

15. Method according to any of claims 1 to 14,
wherein step a) is performed at temperatures below 50°C, particularly below 40°C.

16. Biological heart valve prosthesis, particularly in Transcatheter-Aortic-Valve-Implantation valves, treated by a method according to any of claims 1 to 15.
